# EUROPEAN PATENT SPECIFICATION

(11) **EP 2 861 254 B1**
(45) Date of publication and mention of the grant of the patent: **29.04.2020**
(21) Application number: 13732817.5
(22) Date of filing: 13.06.2013
(51) Int. Cl.: A61K 31/166, A61K 45/06, A61P 9/06, A61K 31/495, A61K 31/4458, A61K 31/444

(54) **PHARMACEUTICAL COMPOSITIONS FOR COMBINATION THERAPY**
PHARMAZEUTISCHE ZUSAMMENSETZUNGEN FÜR EINE KOMBINATIONSTHERAPIE
COMPOSITIONS PHARMACEUTIQUES POUR POLYTHÉRAPIE

(30) Priority: 13.06.2012 DK 201270325
(43) Date of publication of application: 22.04.2015
(73) Proprietor: Acesion Pharma ApS, 2200 Copenhagen N (DK)
(72) Inventor: GRUNNET, Morten, DK-2200 Copenhagen N (DK); SØRENSEN, Ulrik Svane, DK-2200 Copenhagen N (DK); BENTZEN, Bo Hjorth, DK-2200 Copenhagen N (DK); DINESS, Jonas Goldin, DK-2200 Copenhagen N (DK)
(74) Representative: Kjerrumgaard, Lars Bo
(86) International application number: PCT/DK2013/000040
(87) International publication number: WO 2013/185764

(56) References cited:
- WO-A1-2013/104577
- DE-A1- 3 940 315
- J. G. DINESS ET AL: "Inhibition of Small-Conductance Ca2+-Activated K+ Channels Terminates and Protects Against Atrial Fibrillation", CIRCULATION: ARRHYTHMIA AND ELECTROPHYSIOLOGY, vol. 3, no. 4, 19 June 2010 (2010-06-19), pages 380-390, XP055081707, ISSN: 1941-3149, DOI: 10.1161/CIRCEP.110.957407
- PETER M ZYGMUNT ET AL: "Involvement of voltage-dependent potassium channels in the EDHF-mediated relaxation of rat hepatic artery", BRITISH JOURNAL OF PHARMACOLOGY, vol. 121, no. 1, 1 May 1997 (1997-05-01), pages 141-149, XP055081729, ISSN: 0007-1188, DOI: 10.1038/sj.bjp.0701108
- BKAILY GHASSAN ET AL: "Apamin, a highly specific calcium blocking agent in heart muscle", AMERICAN JOURNAL OF PHYSIOLOGY, BETHESDA, MD : AMERICAN PHYSIOLOGICAL SOCIETY, 1898-BALTIMORE, MD. [U.A.] : AMERICAN PHYSIOLOGICAL SOCIETY, US, vol. 248, no. 6, 1 January 1985 (1985-01-01), pages H961-H965, XP009173142, ISSN: 0002-9513
- GU J W ET AL: "Effects of emodin on synaptic transmission in rat hippocampal CA1 pyramidal neurons in vitro", NEUROPHARMACOLOGY, PERGAMON PRESS, OXFORD, GB, vol. 49, no. 1, 1 July 2005 (2005-07-01), pages 103-111, XP027632724, ISSN: 0028-3908 [retrieved on 2005-07-01]
- WANG J ET AL: "Characteristics of spontaneous and evoked GABAergic synaptic currents in cardiac vagal neurons in rats", BRAIN RESEARCH, ELSEVIER, AMSTERDAM, NL, vol. 889, no. 1-2, 19 January 2001 (2001-01-19), pages 78-83, XP027200505, ISSN: 0006-8993 [retrieved on 2001-01-19]
- DINESS JONAS G ET AL: "Negative Modulation of SK Channels Terminates and Protects Against Atrial Fibrillation in Rat and Guinea Pig Ex Vivo and in vivo Models", CIRCULATION, LIPPINCOTT WILLIAMS & WILKINS, US, vol. 120, no. 18, suppl. 2, 1 November 2009 (2009-11-01), page S712, XP009173110, ISSN: 0009-7322
- LEVY ET AL: "Combination therapy for cardiac arrhythmias", AMERICAN JOURNAL OF CARDIOLOGY, CAHNERS PUBLISHING CO., NEWTON, MA, US, vol. 61, no. 2, 15 January 1988 (1988-01-15), pages A95-A100, XP023208837, ISSN: 0002-9149, DOI: 10.1016/0002-9149(88)90749-7 [retrieved on 1988-01-15]
- V CHUBANOV ET AL: "Natural and synthetic modulators of SK (Kca2) potassium channels inhibit magnesium-dependent activity of the kinase-coupled cation channel TRPM7", BRITISH JOURNAL OF PHARMACOLOGY, vol. 166, no. 4, 17 June 2012 (2012-06-17) , pages 1357-1376, XP055081837, ISSN: 0007-1188, DOI: 10.1111/j.1476-5381.2012.01855.x
- J. G. DINESS ET AL: "Inhibition of Small-Conductance Ca2+-Activated K+ Channels Terminates and Protects Against Atrial Fibrillation", CIRCULATION: ARRHYTHMIA AND ELECTROPHYSIOLOGY, vol. 3, no. 4, 19 June 2010 (2010-06-19), pages 380-390, XP055081707, ISSN: 1941-3149, DOI: 10.1161/CIRCEP.110.957407
- PETER M ZYGMUNT ET AL: "Involvement of voltage-dependent potassium channels in the EDHF-mediated relaxation of rat hepatic artery", BRITISH JOURNAL OF PHARMACOLOGY, vol. 121, no. 1, 1 May 1997 (1997-05-01), pages 141-149, XP055081729, ISSN: 0007-1188, DOI: 10.1038/sj.bjp.0701108

## Description

### TECHNICAL FIELD

This invention relates to the use of pharmaceutical compositions comprising a therapeutically effective amount of a sodium channel inhibitor, in a combination with a small-conductance calcium-activated potassium (SK) channel inhibitor, for the treatment of cardiac arrhythmias.

### BACKGROUND ART

The heart is a muscle, which pumps the blood in the circulation by contracting 1-3 times per second. The heartbeat is caused by simultaneous contraction of the individual cardiac muscle cells (cardiac myocytes). The synchronization of the cellular contraction is governed by the electrical cardiac impulse (the cardiac action potential), which is generated in the pacemaker cells of the sine node and spreads rapidly over the heart through a specific conduction system.

Disturbances in the generation of the impulse and the conduction of impulse may occur either as a consequence of a disease, a drug treatment, or electrolyte imbalances. Such disturbances in the impulse are called arrhythmia or dysrythmia and they may lead to unease, emboli, syncope or sudden death. In its simplest form, an arrhythmia covers everything different from a normal cardiac sinus rhythm. Disturbances can cover anything from simple palpitations to devastating ventricular fibrillation including bradycardia and tachycardia.

At a molecular level a group of proteins called ion channels underlie the electrical events in the heart since they are able to conduct electrical currents across the cell membrane. Different types of ion channels are thus instrumental in the generation and conduction of the cardiac action potential, in the regulation of the heart rate by the autonomic nervous system, and in the contractile process in the individual heart cells. The different types of ion channels are therefore evident targets for anti-arrhythmic cardiac drugs, and many anti-arrhythmic drugs on the market do exert their effect by interacting with ion channels.

Anti-arrhythmic drugs are usually divided into four main classes according to the so-called Singh Vaughan Williams classification: Class I compounds all inhibit the cardiac voltage-dependent sodium channel. Some Class I compounds do have additional effects influencing the cardiac action potential being the basis for a further subdivision into three subclasses:
Class IA compounds are sodium channel inhibitors such as Quinidine, Procainamide or Disopyramid, which prolong the action potential;
Class IB compounds are sodium channel inhibitors such as Lidocaine, Mexiletine, Tocainide or Phenytoine, which shorten the action potential; and
Class IC compounds are sodium channel inhibitors such as Flecainide, Moricizine or Propafenone, which do not change the action potential duration.
Class I compounds interact with the sodium channel during its open or inactivated state and are dissociated from the channels during its closed state (during diastole). The rate of dissociation determines whether they show a frequency-dependent channel inhibition. Some of the class I compounds also inhibit subtypes of potassium or calcium permeable channels in addition to their sodium channel inhibiting effect.
Class II compounds are β-adrenoceptor inhibitors and include drugs like Atenolol, Metoprolol, Timolol or Propranolol. β-adrenoceptor inhibitors can be selective for cardiac β1-receptors or have affinity for β1- as well as β2-receptors. Some of the compounds also have an intrinsic β-stimulating effect.
Class III compounds are potassium channel inhibitors such as Amiodarone, Dronedarone, Sotalol, Ibutilide and Dofetilide, which prolong the action potential.
Class IV compounds are inhibitors of L-type calcium channels such as Verapamil.

Small-conductance calcium-activated potassium (SK) channels belongs to the family of Ca²⁺-activated K⁺ channels. Three SK channel subtypes have been cloned: SK1, SK2 and SK3 (corresponding to KCNN1-3 using the genomic nomenclature). The activity of these channels is determined by the concentration of free intracellular calcium ([Ca²⁺]ᵢ) via calmodulin that is constitutively bound to the channels. SK channels are tightly regulated by [Ca²⁺]ᵢ in the physiological range being closed at [Ca²⁺]ᵢ up to around 0.1 µM but fully activated at a [Ca²⁺]ᵢ of 1 µM. Being selective for potassium, open or active SK channels have a hyperpolarizing influence on the membrane potential of the cell. SK channels are widely expressed in the central nervous system (CNS) and in peripheral tissue.

The hyperpolarizing action of active SK channels plays an important role in the control of firing pattern and excitability of excitable cells. SK channel inhibitors such as apamin and *N*-methyl bicuculline, have been demonstrated to increase excitability, whereas the opener, 1-EBIO, is able to reduce electrical activity. In non-excitable cells, where the amount of Ca²⁺ influx via voltage-independent pathways is highly sensitive to the membrane potential, an activation of SK channels will increase the driving force, whereas an inhibitor of SK channels will have a depolarizing effect, and thus diminish the driving force for calcium.

An SK channel inhibitor is a pharmaceutical agent that impairs the conduction of potassium ions (K⁺) through Ca²⁺-activated small conductance K⁺ channels. The impairment can be obtained by any reduction in current resulting from e.g. a direct inhibition of ion conduction to a prevention of Ca²⁺ binding, that is an obligate request for channel activation, or a reduction in calcium sensitivity.

A review of SK channels and SK channel modulators may be found in Wulff H et al.: "Modulators of Small- and Intermediate-Conductance Calcium-Activated Potassium Channels and their Therapeutic Indications", Currrent Medicinal Chemistry 2007 14 1437-1457; and in Liegeois J-F et al.: "Modulation of small conductance calcium-activated potassium (SK) channels: a new challenge in medicinal chemistry", Current Medicinal Chemistry 2003 10 625-647.

Based on the important role of SK channels in linking [Ca²⁺]ᵢ and membrane potential, SK channels are interesting targets for developing novel therapeutic agents, and the potential of inhibitors of SK channels for use in anti-arrhythmic treatment has recently been established, see e.g. Nattel S; J. Physiol. 2009 587 1385-1386; Diness JG, Sørensen US, Nissen JD, Al-Shahib B, Jespersen T, Grunnet M, Hansen RS; Circ. Arrhythm. Electrophysiol. 2010 3 380-90; and Diness et al; Hypertension 2011 57 1129-1135.

WO 2006/013210 describes certain 2-amino benzimidazole derivatives and their use as modulators of small-conductance calcium-activated potassium channels.

### SUMMARY OF THE INVENTION

The present invention is based on the discovery that a combination of two different antiarrhythmic drugs, belonging to separate classes, and representing different mode of actions, offer certain advantages when compared to conventional mono therapy of either of the compounds. The beneficial effect can be ascribed to the possibility for achieving a therapeutic effect when administering a dose or concentration of the two antiarrhythmic drugs that would not give a therapeutic effect if either of the drugs were administered in the same dose as mono therapy. A beneficial effect can also arise if one or both of the drugs are administered in a therapeutic dose and the therapeutic effect then is larger than the predicted additive effect expected if they are administered in mono-therapy. In the case where a therapeutic effect is obtained with lower doses than would otherwise be required to obtain a similar effect, the window towards adverse effects will be increased. This would be an important benefit as many of the current antiarrhythmic drugs have risk of severe dose related side effects.

Therefore, in its first aspect, the invention provides a combination of
(i) a small-conductance calcium-activated potassium (SK) channel inhibitor, or a pharmaceutically acceptable salt or solvate thereof; and
(ii) a compound selected from
   *N*-(2,6-dimethylphenyl)-2-[4-[2-hydroxy-3-(2-methoxyphenoxy)-propyl]piperazin-1-yl]acetamide,
   (3*R*)-1-{(1*R*,2*R*)-2-[2-(3,4-dimethoxyphenyl)ethoxy]cyclohexyl}pyrrolidin-3-ol,
   1-[2-[bis(4-fluorophenyl)methoxy]ethyl]-4-(3-phenylpropyl)piperazine,
   2-butyl-3-benzofuranyl 4-[2-(diethylamino)-ethoxy]-3,5diiodophenyl ketone,
   N-{2-butyl-3-[4-(3-dibutylaminopropoxy)benzoyl]benzofuran-5-yl} methanesulfonamide,
   N-[4-[2-[2-[4-(methanesulfonamido)phenoxy]ethyl-methylamino]ethyl]phenyl]-methanesulfonamide,
   N-[4-[4-[ethyl(heptyl)amino]-1-hydroxybutyl]phenyl]methanesulfonamide,
   N-[4-[1-hydroxy-2-(propan-2-ylamino)ethyl]phenyl]methanesulfonamide, or a pharmaceutically acceptable salt or solvate thereof,
   for use as a medicament.

In one embodiment the compound is selected from Ranolazine dihydrochloride, Vernakalant hydrochloride, Vanoxerine dihydrochloride, Vanoxerine hydrochloride, Amiodarone hydrochloride, Dronedarone hydrochloride, Dofetilide, Ibutilide fumarate, and Sotalol hydrochloride.

In a further embodiment the compound is Ranolazine dihydrochloride.

In a still further embodiment, the small-conductance calcium-activated potassium (SK) channel inhibitor is a SK negative modulator. In another embodiment the small-conductance calcium-activated potassium (SK) channel inhibitor is a SK channel pore blocker.

In a second aspect, the invention concerns a pharmaceutical composition comprising the combination according to any one of claims 1-5.

In a third aspect, the invention concerns the combination according to any one of claims 1-5 for use as a medicament for the treatment of a cardiac disease, disorder or condition associated with an abnormal rhythm of the heart selected from cardiac arrhythmia, atrial arrhythmia, ventricular arrhythmia, atrial fibrillation, ventricular fibrillation, tachyarrhythmia, atrial tachyarrhythmia, ventricular tachyarrhythmia, and bradyarrhythmias. In an embodiment the cardiac disease, disorder or condition associated with an abnormal rhythm of the heart is atrial fibrillation. In a more specific embodiment the combination is useful for treatment of atrial fibrillation by acute cardioversion to normal sinus rhythm. In another embodiment the combination is useful for treatment of atrial fibrillation by maintaining normal sinus rhythm and avoiding or reducing the occurrence of new episodes of atrial fibrillation.

In a fourth aspect the present invention relates to a kit of parts comprising at least two separate unit dosage forms (A) and (B), wherein
(A) comprises a compound selected from
   *N*-(2,6-dimethylphenyl)-2-[4-[2-hydroxy-3-(2-methoxyphenoxy)-propyl]piperazin-1-yl]acetamide,
   (3*R*)-1-{(1*R*,2*R*)-2-[2-(3,4-dimethoxyphenyl)ethoxy]cyclohexyl}pyrrolidin-3-ol,
   1-[2-[bis(4-fluorophenyl)methoxy]ethyl]-4-(3-phenylpropyl)piperazine,
   2-butyl-3-benzofuranyl 4-[2-(diethylamino)-ethoxy]-3,5diiodophenyl ketone,
   N-{2-butyl-3-[4-(3-dibutylaminopropoxy)benzoyl]benzofuran-5-yl} methanesulfonamide,
   N-[4-[2-[2-[4-(methanesulfonamido)phenoxy]ethyl-methylamino]ethyl]phenyl]-methanesulfonamide,
   N-[4-[4-[ethyl(heptyl)amino]-1-hydroxybutyl]phenyl]methanesulfonamide, and
   N-[4-[1-hydroxy-2-(propan-2-ylamino)ethyl]phenyl]methanesulfonamide;
   and
(B) comprises a small-conductance calcium-activated potassium (SK) channel inhibitor, or a pharmaceutically acceptable salt or solvate thereof; and optionally
(C) instructions for the simultaneous, sequential or separate administration of the compound of (A) and the small-conductance calcium-activated potassium (SK) channel inhibitor of (B), to a patient in need thereof.

Other objects of the invention will be apparent to the person skilled in the art from the following detailed description and examples.

### DETAILED DISCLOSURE OF THE INVENTION

### Definitions

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. The patient to be treated is preferably a mammal; in particular a human being, but it may also include animals, such as dogs, cats, cows, horses, sheep and pigs.

In the present context, the term "pharmaceutically acceptable salt" is intended to indicate salts which are not harmful to the patient. Such salts include pharmaceutically acceptable acid addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like. Further examples of pharmaceutically acceptable inorganic or organic acid addition salts include the pharmaceutically acceptable salts listed in J. Pharm. Sci. 66, 2, (1977) which is incorporated herein by reference. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like.

In the present context, the term "a solvate" is intended to mean solvates as understood by a person skilled in the art, such solvates may without limitation be selected from organic solvents and inorganic solvents, such as water, e.g. a mono- or hemihydrate.

When reference is made to a pharmaceutically acceptable salt or solvate thereof it is understood to include a compound as a salt, a compound as a solvate and a compound as both a salt and a solvate.

A "therapeutically effective amount" of a compound or combination of compounds as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

When an "effective amount" is used herein, it is intended to mean an amount of a compound which is lower than the therapeutically effective amount, but where the effective amount of a compound in combination with the effective amount of another compound as described herein means that the combined amounts leads to a therapeutically effective amount.

Administration of pharmaceutical compositions according to the invention may be through several routes of administration, for example, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, parenteral, e.g. intravenous, or via an impregnated or coated device such as a stent, etc to patients in need of such a treatment.

When administered separately, the SK channel inhibitor may be administered before the other antiarrhythmic drug substance or alternatively the latter may be administered before the SK channel inhibitor. Alternatively, the SK channel inhibitor and the other antiarrhythmic drug substance may be administered at the same time.

Compositions of the current invention may be administered in several dosage forms, for example, as solutions, suspensions, emulsions, microemulsions, multiple emulsion, foams, salves, pastes, plasters, ointments, tablets, coated tablets, rinses, capsules, for example, hard gelatine capsules and soft gelatine capsules, suppositories, rectal capsules, drops, gels, sprays, powder, aerosols, inhalants, eye drops, ophthalmic ointments, ophthalmic rinses, vaginal pessaries, vaginal rings, vaginal ointments, injection solution, in situ transforming solutions, for example in situ gelling, in situ setting, in situ precipitating, in situ crystallization, infusion solution, and implants.

Compositions of the current invention are useful in the composition of solids, semi-solids, powder and solutions for pulmonary administration of the compounds for use of the invention, using, for example a metered dose inhaler, dry powder inhaler and a nebulizer, all being devices well known to those skilled in the art.

Parenteral administration may be performed by subcutaneous, intramuscular, intraperitoneal or intravenous injection by means of a syringe, optionally a penlike syringe. Alternatively, parenteral administration can be performed by means of an infusion pump. A further option is a composition which may be a solution or suspension for the administration of the compounds for use of the invention in the form of a nasal or pulmonal spray. As a still further option, the pharmaceutical compositions containing the compounds for use of the invention of the invention can also be adapted to transdermal administration, e.g. by needle-free injection or from a patch, optionally an iontophoretic patch, or transmucosal, e.g. buccal, administration.

In the present context the term " adjuvants, excipients, carriers and/or diluents" is understood by a person skilled in the art of making pharmaceutical or veterinary compositions and means any material, which is inert in the sense that it substantially does not have any therapeutic and/or prophylactic effect per se. Such adjuvants, excipients, carriers and/or diluents may be added with the purpose of making it possible to obtain a pharmaceutical composition, which has acceptable technical properties.

### Sodium Channel Blocking Agents

Sodium channel inhibitors are pharmaceutical agents that impair the conduction of sodium ions (Na⁺) through sodium channels. The impairment can be obtained by reduction in peak current, a reduction in late current or a combination of both. Further, the inhibition can exert use-dependency being more effective at high frequency exemplified by being most efficacious at high cardiac beating frequency or by reverse use-dependency being most efficacious at low cardiac beating frequency. Inhibitors can be pore blockers (referred herein as to a sodium channel pore blocker) or negative modulators (referred herein as to a sodium channel negative modulator) and binding kinetic to the channel can exhibit fast or slow on and off rates, a phenomenon that has guided the traditional sub-division of cardiac class I anti-arrhythmic agents. Na+ channel inhibitors can also be state dependent with physiological efficacy relying on membrane potential and inter time interval between concomitant action potentials (referred herein as to a state dependent sodium channel inhibitor).

In an embodiment of any one of the aspects as described above the sodium channel inhibitor, or a pharmaceutically acceptable salt or solvate thereof is selected from sodium channel pore blockers, sodium channel negative modulators, or state dependent sodium channel inhibitors.

Such sodium channel inhibitor may be selected from is a Class IA antiarrhythmic drug, a Class IB antiarrhythmic drug, or a Class IC antiarrhythmic drug.

### Other useful antiarrhythmic drug

In addition to the above described Class 1A, 1B, and 1C antiarrhythmic drugs useful in combination with a small-conductance calcium-activated potassium (SK) channel inhibitor, or a pharmaceutically acceptable salt or solvate thereof as described herein, some additional antiarrhythmic drugs are believed to have beneficial properties similar to those disclosed for the Class 1A, 1B, and 1C antiarrhythmic drugs, in combination with a small-conductance calcium-activated potassium (SK) channel inhibitor, or a pharmaceutically acceptable salt or solvate thereof as described herein.

Suitable antiarrhythmic drugs are
Ranolazine dihydrochloride
*N*-(2,6-dimethylphenyl)-2-[4-[2-hydroxy-3-(2-methoxyphenoxy)-propyl]piperazin-1-yl]acetamide dihydrochloride;
Vernakalant hydrochloride
(3*R*)-1-{(1*R*,2*R*)-2-[2-(3,4-dimethoxyphenyl)ethoxy]cyclohexyl)pyrrolidin-3-ol hydrochloride;
Vanoxerine dihydrochloride
1-[2-[bis(4-fluorophenyl)methoxy]ethyl]-4-(3-phenylpropyl)piperazine dihydrochloride; Vanoxerine hydrochloride
1-[2-[bis(4-fluorophenyl)methoxy]ethyl]-4-(3-phenylpropyl)piperazine hydrochloride; Amiodarone hydrochloride
2-butyl-3-benzofuranyl 4-[2-(diethylamino)-ethoxy]-3,5diiodophenyl ketone hydrochloride;
Dronedarone hydrochloride
N-{2-butyl-3-[4-(3-dibutylaminopropoxy)benzoyl]benzofuran-5-yl} methanesulfonamide hydrochloride;
Dofetilide
N-[4-[2-[2-[4-(methanesulfonamido)phenoxy]ethyl-methylamino]ethyl]phenyl]methanesulfonamide;
Ibutilide fumarate
N-[4-[4-[ethyl(heptyl)amino]-1-hydroxybutyl]phenyl]methanesulfonamide fumarate; and Sotalol hydrochloride
N-[4-[1-hydroxy-2-(propan-2-ylamino)ethyl]phenyl]methanesulfonamidehydrochloride.

Thus, in a further embodiment a small-conductance calcium-activated potassium (SK) channel inhibitor, or a pharmaceutically acceptable salt or solvate thereof is combined with an antiarrhythmic drug selected from *N*-(2,6-dimethylphenyl)-2-[4-[2-hydroxy-3-(2-methoxyphenoxy)-propyl]piperazin-1-yl]acetamide;
or a pharmaceutically acceptable salt or solvate thereof.

In a still further embodiment the antiarrhythmic drug is selected from (3*R*)-1-{(1*R*,2*R*)-2-[2-(3,4-dimethoxyphenyl)ethoxy]cyclohexyl}pyrrolidin-3-ol; or a pharmaceutically acceptable salt or solvate thereof.

In a further embodiment the antiarrhythmic drug is selected from 1-[2-[bis(4-fluorophenyl)methoxy]ethyl]-4-(3-phenylpropyl)piperazine; or a pharmaceutically acceptable salt or solvate thereof.

In a still further embodiment the antiarrhythmic drug is selected from 2-butyl-3-benzofuranyl 4-[2-(diethylamino)-ethoxy]-3,5diiodophenyl ketone; or a pharmaceutically acceptable salt or solvate thereof.

In a further embodiment the antiarrhythmic drug is selected from N-{2-butyl-3-[4-(3-dibutylaminopropoxy)benzoyl]benzofuran-5-yl} methanesulfonamide; or a pharmaceutically acceptable salt or solvate thereof.

In a still further embodiment the antiarrhythmic drug is selected from N-[4-[2-[2-[4-(methanesulfonamido)phenoxy]ethyl-methylamino]ethyl]phenyl]-methanesulfonamide;
or a pharmaceutically acceptable salt or solvate thereof.

In a further embodiment the antiarrhythmic drug is selected from N-[4-[4-[ethyl(heptyl)amino]-1-hydroxybutyl]phenyl]methanesulfonamide fumarate; or a pharmaceutically acceptable salt or solvate thereof.

In a still further embodiment the antiarrhythmic drug is selected from N-[4-[1-hydroxy-2-(propan-2-ylamino)ethyl]phenyl]methanesulfonamidehydrochloride; or a pharmaceutically acceptable salt or solvate thereof.

### SK Blocking Agents

An SK channel inhibitor is a pharmaceutical agent that impairs the conduction of potassium ions (K⁺) through Ca²⁺-activated small conductance K⁺ channels of any of the subtypes SK1, SK2 or SK3 channels encoded for by the genes *KCNN1, KCNN2* or *KCNN3* . The impairment can be obtained by any reduction in current resulting from a direct inhibition of ion conduction to a prevention of Ca²⁺ binding, that is an obligate request for channel activation (SK channel pore block), or a reduction in calcium sensitivity. A direct inhibition is exemplified by a pore blocker (referred herein to as a SK channel pore blocker) that interacts with the pore of the SK channel to a degree that prevents ion conduction through the channel. Pore blockers can belong to classes of toxins, peptides or small organic molecules. A compound that reduce the calcium sensitivity of the SK channel exert its mode of action by reducing or preventing the ability of Ca²⁺ to bind to calmodulin that is bound to SK channels. Since binding of Ca²⁺ to calmodulin that is associated to SK channels is a prerequisite for SK channel activation, compounds that reduce or prevent this binding is described as negative modulators (referred herein to as a SK negative modulator). Further their effect can be either direct interference with the Ca²⁺ binding or indirect via negative allosteric interactions.

In an embodiment the small-conductance calcium-activated potassium (SK) channel inhibitor is selected from a SK channel pore blocker. Typical examples of such pore blockers are selected from N-(pyridin-2-yl)-4-(pyridin-2-yl)thiazol-2-amine, apamin, scyllatoxin, leuurotoxin, tubocurarine, meurotoxin, dequalinium, bicuculline, and 6,12,19,20,25,26-Hexahydro-5,27:13,18:21,24-trietheno-11,7-metheno-7*H*-dibenzo [*b*,*n*]-[1,5,12,16]tetraazacyclotricosine-5,13-diium (UCL-1684) or a pharmaceutically acceptable salt or solvate thereof. Each of these pore blockers constitutes individual embodiments and may be combined with any one of the above described antiarrhythmic drugs.

In a further embodiment the small-conductance calcium-activated potassium (SK) channel inhibitor is selected from a SK negative modulator. Typical example of such modulator is (*R*)-*N*-(benzimidazol-2-yl)-1,2,3,4-tetrahydro-1-naphtylamine or a pharmaceutically acceptable salt or solvate thereof. This negative modulator may be combined with any one of the above described antiarrhythmic drugs.

### Cardiac Diseases

In the context of this invention a cardiac disease, disorder or condition is any cardiac disease, disorder or condition, including, but not limited to, an abnormal rhythm of the heart or variant and exercise induced angina.

In a more specific embodiment the cardiac disease, disorder or condition is any disease, disorder or condition associated with an abnormal rhythm of the heart or variant and exercise induced angina.

In a more specific embodiment the cardiac disease, disorder or condition is any disease, disorder or condition associated with an abnormal rhythm of the heart.

In a more specific embodiment the cardiac disease, disorder or condition associated with an abnormal rhythm of the heart is selected from cardiac arrhythmia, atrial arrhythmia, ventricular arrhythmia, atrial fibrillation, ventricular fibrillation, tachyarrhythmia, atrial tachyarrhythmia, ventricular tachyarrhythmia, and bradyarrhythmias.

In another embodiment a cardiac disease, disorder or condition of the invention is an abnormal rhythm caused by myocardial ischaemia, myocardial infarction, cardiac hypertrophy, or cardiomyopathy.

In a further specific embodiment the cardiac disease, disorder or condition associated with an abnormal rhythm of the heart is a cardiac arrhythmia caused by a genetic disease.

In a still further preferred embodiment the cardiac disease, disorder or condition associated with an abnormal rhythm of the heart is cardiac arrhythmia.

In a preferred embodiment the cardiac disease, disorder or condition associated with an abnormal rhythm of the heart is atrial fibrillation.

In a particular embodiment the combination of the present invention is useful for treatment of atrial fibrillation by acute cardioversion to normal sinus rhythm.

In another particular embodiment the combination of the present invention is useful for treatment of atrial fibrillation by maintaining normal sinus rhythm and avoiding or reducing the occurrence of new episodes of atrial fibrillation.

### Pharmacological Treatment of atrial fibrillation

In the context of this invention and as understood by a person skilled in the art treatment of atrial fibrillation is acute cardioversion or maintenance of sinus rhythm or both. Acute conversion is defined as application of compound that has the ability to convert atrial fibrillation to a normal cardiac sinus rhythm. Normal sinus rhythm is defined as regular stable heart beating at frequencies between 40 and 100 beats at rest in adults with normal regular p-wave on a standard 12-lead electrocardiogram. Maintenance of sinus rhythm is defined as the ability for a compound to preserve a normal stable sinus rhythm over time with no relapse to atrial fibrillation or the ability of a compound to significantly reduced the incidence of relapse from atrial fibrillation to normal sinus rhythm compared to non-treated controls.

Further embodiments of the combination of the present invention are described in the experimental section herein, and each individual combination as well as each pharmaceutical composition comprising such combinations, kit of parts, article of manufacture, uses and methods constitutes embodiments that may form part of claims.

The above embodiments should be seen as referring to any one of the aspects (such as 'method for treatment', 'use of', 'combinations', 'pharmaceutical compositions', article of manufacture, and 'kit of parts') described herein as well as any one of the embodiments described herein unless it is specified that an embodiment relates to a certain aspect or aspects of the present invention.

All headings and sub-headings are used herein for convenience only.

Any combination of the above-described elements in all possible variations thereof is encompassed by the invention unless otherwise indicated herein or otherwise clearly contradicted by context.

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless other-wise indicated herein, and each separate value is incorporated into the specification as if it were individually recited herein. Unless otherwise stated, all exact values provided herein are representative of corresponding approximate values (e.g., all exact exemplary values provided with respect to a particular factor or measurement can be considered to also pro-vide a corresponding approximate measurement, modified by "about," where appropriate).

All methods described herein can be performed in any suitable order unless other-wise indicated herein or otherwise clearly contradicted by context.

The use of any and all examples, or exemplary language (e.g., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents.

The description herein of any aspect or embodiment of the invention using terms such as "comprising", "having", "including" or "containing" with reference to an element or elements is intended to provide support for a similar aspect or embodiment of the invention that "consists of", "consists essentially of, or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

The present invention is further illustrated by the following examples. The features disclosed in the foregoing description and in the following examples may, both separately and in any combination thereof, be material for realizing the invention in diverse forms thereof.

### Experimental

### Methods

All of the studies were performed under a license from the Danish Ministry of justice (license No 2012/15-2934-00345) and in accordance with the Danish guidelines for animal experiments according to the European Commission Directive 86/609/EEC.

### Langendorff preparation:

The isolated heart was prepared as follows. Guinea pigs weighing between 350 and 600 g were anesthetized with i.p. injection of 200 mg/kg pentobarbital. In addition, 1000 IU/kg heparin were injected i.p. Respiration was maintained by artificial ventilation (atm air) through a cannula in the trachea (volume, 12 ml/kg; rate, 60 strokes/min). Upon thoracotomy, a perfusion cannula was inserted and fixed in the aorta for retrograde perfusion. Hearts were mounted in a vertical Langendorff set-up (Hugo Sachs-Harvard Apparatus GmbH, March-Hugstetten, Germany) and perfused with Krebs-Henseleit solution: 120.4 mM NaCl, 4.0 mM KCl, 2.5 mM CaCl₂, 0.6 mM MgSO₄, 15.3 mM NaHCO₃, 0.6 mM NaH₂PO₄, and 11.5 mM glucose, pH 7.4, at constant oxygenation with O₂/CO₂ in a 95/5% mixture at 37°C. Four monophasic action potential (MAP) electrodes were placed on the left atrium, the right atrium, the left ventricle, and right ventricle, respectively. A pacing electrode was placed on the right atrium. The electrocardiogram (ECG) was obtained with three ECG electrodes placed in close proximity to the heart. All data were acquired using the 16-channel PowerLab system (ADlnstruments, Oxfordshire, UK). Pacing stimuli were twice the diastolic threshold and with a duration of 2 milliseconds (ms) unless otherwise stated.

### Dose finding:

Isolated, perfused female guinea pig hearts from Dunkin Hartley strain (HsdPoc:DH) continuously paced on the right atrium (300 bpm) were allowed to stabilize, and the atrial effective refractory period (aERP) was measured. AF was induced by 1 µmol/L acetylcholine (ACh) combined with burst pacing (50 Hz, 10V). When sustained (>3 minutes) AF was obtained, the test compound from a stock concentration of 10 mMol/L in DMSO was added to the perfusate in a concentration of 100 nmol/L for 30 min. If no cardioversion was obtained within 30 minutes, the concentration of the compound in the perfusate was increased to 300 nmol/L. If no cardioversion was obtained within an additional time period of 30 minutes, the concentration of the compound in the perfusate was increased to 1.0 µmol/L. If no cardioversion was obtained within a further time period of 30 minutes, the concentration of the compound in the perfusate was increased to 3.0 µmol/L. If no cardioversion was obtained within an additional time period of 30 minutes, the concentration of the compound in the perfusate was increased to 10.0 µmol/L. If no cardioversion was obtained within an additional time period of 30 minutes, the concentration of the compound in the perfusate was increased to 30.0 µmol/L. Cardioversion was considered obtained if sinus rhythm was obtained within 30 minutes of one of the above mentioned drug applications, and if sustained AF could not be reinduced with burst pacing stimulation immediately after this.

### Determination of effects on aERP and AF duration:

Isolated, perfused female guinea pig hearts from Dunkin Hartley strain (HsdPoc:DH) continuously paced on the RA at a rate of 300 bpm were allowed to stabilize and perfused for 30 minutes with the highest concentration of test compound that had failed to achieve cardioversion as described in the 'dose finding' section above. The aERP was measured (10 basic stimuli [called S1] followed by premature S2 stimuli applied with 2-ms decrements) immediately before and at the end of the 30-minute period at pacing rates of 5 Hz and 10 Hz. After the 30 minutes of perfusion with either 100 nmol/L N-(pyridin-2-yl)-4-(pyridin-2-yl)thiazol-2-amine (ICA), 1 µmol/L flecainide, or 3 µmol/L ranolazine, or vehicle, 1 µmol/L ACh was added to the perfusate and induction of AF was attempted 40 times with burst-pacing (50 Hz) for 2 seconds or until sustained (duration >3 min) AF was obtained (test compound dissolved in DMSO in a stock concentration of 10 mMol/L). ICA in a concentration of 100 nmol/L was then tested in combination with either 1 µmol/L flecainide or 3 µmol/L ranolazine using the same protocol as described above in this section.

### Results

### Dose finding:

In the dose finding study, cardioversion was obtained as presented in Table 1. To clarify, for example '4/4' for ranolazine in a concentration of 0.3 µM means that conversion was obtained in four out of four animals in the given concentration of test compound.

**Table 1**

| Concentration | 0.1 µM | 0.3 µM | 1.0 µM | 3.0 µM | 10.0 µM | 30.0 µM | No cardioversion |
|---|---|---|---|---|---|---|---|
| ICA | | 4/4 | | | | | |
| Flecainide | | | | 2/4 | 2/4 | | |
| Ranolazine | | | 1/5 | 2/5 | | | 2/5 |

On basis of these findings, concentrations of 100 nmol/L ICA, 1.0 µmol/L flecainide, and 3 µmol/L ranolazine were chosen to study effects on aERP and AF duration as described in the section above.

### Effects on aERP:

The effects on aERP are presented in Table 2 as the percentual prolongation of aERP after 30 minutes of perfusion with test compound(s) as compared to the baseline. Values are given in % ± SEM:
In the table, * denote statistical significance as compared to ICA administered alone, ¤ denote statistical significance as compared to flecainide administered alone, and # denote statistical significance as compared to ranolazine administered alone.

**Table 2**

| Pacing frequency | 5 Hz | 10 Hz |
|---|---|---|
| Time matched control | 105.4±5.2 | 98.4±5.1 |
| ICA (0.1 µM) | 112.5±3.8 | 119.3±10.6 |
| Flecainide (1 µM) | 123.0±2.6 | 124.6±8.0 |
| Ranolazine (3 µM) | 114.81±12.7 | 114.4±9.9 |
| ICA (0.1 µM) + flecainide (1 µM) | 152.7±17.4* | 188.6±28.5 *, ¤ |
| ICA (0.1 µM) + ranolazine (3 µM) | 115.9±13.8 | 118.5±20.7 |

| | | |
|---|---|---|
| * Statistically significant compared to ICA administered alone ¤ Statistically significant compared to flecainide administered alone | | |

In the doses used, ICA, flecainide, and ranolazine caused no significant prolongation of aERP from baseline values as compared to time matched controls when they were administered alone. In contrast, the combination of the same concentrations of ICA and flecainide produced a prolongation of aERP that was significantly greater than for either compound alone at 10 Hz and a prolongation of aERP that was significantly greater than that of ICA alone at 5 Hz. The combination of ICA and ranolazine did not cause a prolongation of aERP that was greater than for any of the compounds alone neither at 5 Hz nor 10 Hz.

### Effects on AF duration:

The effects on the log(10)-transformed AF durations are presented in Table 3 and given as means ± SEM.

| | Log AF duration |
|---|---|
| Time matched control | 1.43±0.06 |
| ICA (0.1 µM) | 1.29±0.15 |
| Flecainide (1 µM) | 1.27±0.14 |
| Ranolazine (3 µM) | 1.11±0.23 |
| ICA (0.1 µM) + flecainide (1 µM) | 0.45±0.09 *, ¤ |
| ICA (0.1 µM) + ranolazine (3 µM) | -0.81±52 *, # |

| | |
|---|---|
| * Statistically significant compared to ICA administered alone ¤ Statistically significant compared to flecainide administered alone # Statistically significant compared to ranolazine administered alone | |

In the doses used, ICA, flecainide, and ranolazine caused no significant reduction of AF duration as compared to time matched controls when they were administered alone. The combination of ICA and flecainide produced a reduction of AF duration that was significantly greater than for either compound alone. Likewise, the combination of ICA and ranolazine produced a reduction of AF duration that was significantly greater than for either compound alone.

### Conclusion

The SK blocker ICA does not cause any significant effects on aERP or AF duration in a concentration of 100 nMol/L in the models described above. Likewise, the concentrations of the sodium channel blockers flecainide (1.0 µMol/L) and ranolazine (3 µMol/L) were sub-effective on both aERP and AF duration when applied alone. However, the SK channel blocker ICA co-applied with a sodium channel blocker, both in concentrations that were sub-effective when administered alone, caused significant reductions of aERP and AF durations when combined. Thus, we have demonstrated the principle that the combination of an SK channel inhibitor and a sodium channel blockers act synergistically to increase aERP and reduce AF duration in concentrations that for the individual drug substance is without effect. The data obtained thereby support the claimed principle for obtaining antiarrhythmic effects by combining an SK channel inhibitor with an antiarrhythmic drug from another class.

## Claims

1. A combination of
(i) a small-conductance calcium-activated potassium (SK) channel inhibitor, or a pharmaceutically acceptable salt or solvate thereof; and
(ii) a compound selected from
*N*-(2,6-dimethylphenyl)-2-[4-[2-hydroxy-3-(2-methoxyphenoxy)-propyl]piperazin-1-yl]acetamide,
(3*R*)-1-{(1*R*,2*R*)-2-[2-(3,4-dimethoxyphenyl)ethoxy]cyclohexyl}pyrrolidin-3-ol,
1-[2-[bis(4-fluorophenyl)methoxy]ethyl]-4-(3-phenylpropyl)piperazine,
2-butyl-3-benzofuranyl 4-[2-(diethylamino)-ethoxy]-3,5diiodophenyl ketone,
N-{2-butyl-3-[4-(3-dibutylaminopropoxy)benzoyl]benzofuran-5-yl} methanesulfonamide,
N-[4-[2-[2-[4-(methanesulfonamido)phenoxy]ethyl-methylamino]ethyl]phenyl]-methanesulfonamide,
N-[4-[4-[ethyl(heptyl)amino]-1-hydroxybutyl]phenyl]methanesulfonamide,
N-[4-[1-hydroxy-2-(propan-2-ylamino)ethyl]phenyl]methanesulfonamide,or a pharmaceutically acceptable salt or solvate thereof,
for use as a medicament.

2. The combination for use according to claim 1 wherein the compound is selected from Ranolazine dihydrochloride, Vernakalant hydrochloride, Vanoxerine dihydrochloride, Vanoxerine hydrochloride, Amiodarone hydrochloride, Dronedarone hydrochloride, Dofetilide, Ibutilide fumarate, and Sotalol hydrochloride.

3. The combination for use according to any one of claims 1-2 wherein the compound is Ranolazine dihydrochloride.

4. The combination for use according to any one of claims 1-3, wherein the small-conductance calcium-activated potassium (SK) channel inhibitor is a SK negative modulator.

5. The combination for use according to any one of claims 1-3, wherein the small-conductance calcium-activated potassium (SK) channel inhibitor is a SK channel pore blocker.

6. A pharmaceutical composition comprising the combination according to any one of claims 1-5.

7. The combination according to any one of claims 1-5 for use as a medicament for the treatment of a cardiac disease, disorder or condition associated with an abnormal rhythm of the heart selected from cardiac arrhythmia, atrial arrhythmia, ventricular arrhythmia, atrial fibrillation, ventricular fibrillation, tachyarrhythmia, atrial tachyarrhythmia, ventricular tachyarrhythmia, and bradyarrhythmias.

8. The combination for use according to claim 7, wherein the cardiac disease, disorder or condition associated with an abnormal rhythm of the heart is atrial fibrillation.

9. The combination for use according to claim 8 useful for treatment of atrial fibrillation by acute cardioversion to normal sinus rhythm.

10. The combination for use according to claim 8 useful for treatment of atrial fibrillation by maintaining normal sinus rhythm and avoiding or reducing the occurrence of new episodes of atrial fibrillation.

11. A kit of parts comprising at least two separate unit dosage forms (A) and (B), wherein
(A) comprises a compound selected from
*N*-(2,6-dimethylphenyl)-2-[4-[2-hydroxy-3-(2-methoxyphenoxy)-propyl]piperazin-1-yl]acetamide,
(3*R*)-1-{(1*R*,2*R*)-2-[2-(3,4-dimethoxyphenyl)ethoxy]cyclohexyl}pyrrolidin-3-ol,
1-[2-[bis(4-fluorophenyl)methoxy]ethyl]-4-(3-phenylpropyl)piperazine,
2-butyl-3-benzofuranyl 4-[2-(diethylamino)-ethoxy]-3,5diiodophenyl ketone,
N-{2-butyl-3-[4-(3-dibutylaminopropoxy)benzoyl]benzofuran-5-yl} methanesulfonamide,
N-[4-[2-[2-[4-(methanesulfonamido)phenoxy]ethyl-methylamino]ethyl]phenyl]-methanesulfonamide,
N-[4-[4-[ethyl(heptyl)amino]-1-hydroxybutyl]phenyl]methanesulfonamide, and
N-[4-[1-hydroxy-2-(propan-2-ylamino)ethyl]phenyl]methanesulfonamide;
and
(B) comprises a small-conductance calcium-activated potassium (SK) channel inhibitor, or a pharmaceutically acceptable salt or solvate thereof; and optionally
(C) instructions for the simultaneous, sequential or separate administration of the compound of (A) and the small-conductance calcium-activated potassium (SK) channel inhibitor of (B), to a patient in need thereof.

## Patentansprüche

1. Eine Kombination von
(i) einem Hemmer der SK-Kanäle (Calcium-aktivierten Kalium-Kanäle mit kleiner Leitfähigkeit)
oder einem pharmazeutisch akzeptablen Salz oder Solvat davon; und
(ii) einer Verbindung, die ausgewählt ist aus
*N*-(2,6-Dimethylphenyl)-2-[4-[2-hydroxy-3-(2-methoxyphenoxy)-propyl]piperazin-1-yl]acetamid,
(3*R*)-1-{(1*R*,2*R*)-2-[2-(3,4-Dimethoxyphenyl)ethoxy]cyclohexyl}pyrrolidin-3-ol,
1-[2-[Bis(4-fluorophenyl)methoxy]ethyl]-4-(3-phenylpropyl)piperazin,
2-Butyl-3-benzofuranyl-4-[2-(diethylamino)-ethoxy]-3,5-diiodphenylketon,
N-{2-Butyl-3-[4-(3-dibutylaminopropoxy)benzoyl]benzofuran-5-yl}methansulfonamid,
N-[4-[2-[2-[4-(Methansulfonamido)phenoxy]ethyl-methylamino]ethyl]phenyl]-methansulfonamid,
N-[4-[4-[Ethyl(heptyl)amino]-1-hydroxybutyl]phenyl]methansulfonamid,
N-[4-[1-Hydroxy-2-(propan-2-ylamino)ethyl]phenyl]methansulfonamid, oder einem pharmazeutisch akzeptablen Salz oder Solvat davon,
zur Verwendung als Arzneimittel.

2. Die Kombination zur Verwendung nach Anspruch 1, wobei die Verbindung ausgewählt ist aus Ranolazindihydrochlorid, Vernakalanthydrochlorid, Vanoxerindihydrochlorid, Vanoxerinhydrochlorid, Amiodaronhydrochlorid, Dronedaronhydrochlorid, Dofetilid, Ibutilidfumarat und Sotalolhydrochlorid.

3. Die Kombination zur Verwendung nach einem der Ansprüche 1-2, wobei die Verbindung Ranolazindihydrochlorid ist.

4. Die Kombination zur Verwendung nach einem der Ansprüche 1-3, wobei der Hemmer der SK-Kanäle (Calcium-aktivierten Kalium-Kanäle mit kleiner Leitfähigkeit) ein negativer SK-Modulator ist.

5. Die Kombination zur Verwendung nach einem der Ansprüche 1-3, wobei der Hemmer der SK-Kanäle (Calcium-aktivierten Kalium-Kanäle mit kleiner Leitfähigkeit) ein SK-Kanalporenblocker ist.

6. Eine pharmazeutische Zusammensetzung umfassend die Kombination nach einem der Ansprüche 1-5.

7. Die Kombination nach einem der Ansprüche 1-5 zur Verwendung als Arzneimittel zur Behandlung von einer Herzerkrankung, einer Herzstörung oder eines Herzleidens, die bzw. das in Zusammenhang mit einem abnormalen Rhythmus des Herzens ausgewählt aus Herzarrhythmie, atrialer Arhythmie, ventrikulärer Arhythmie, Vorhofflimmern, ventrikulärer Fibrillation, Tachyarrhythmie, atrialer Tachyarrhythmie, ventrikulärer Tachyarrhythmie und Bradyarrhythmie steht.

8. Die Kombination zur Verwendung nach Anspruch 7, wobei die Herzerkrankung, die Herzstörung oder das Herzleiden, die bzw. das in Zusammenhang mit einem abnormalen Rhythmus des Herzens steht Vorhofflimmern ist.

9. Die Kombination zur Verwendung nach Anspruch 8, die zur Behandlung des Vorhofflimmerns durch akute Kardioversion in den normalen Sinusrhythmus nützlich ist.

10. Die Kombination zur Verwendung nach Anspruch 8, die zur Behandlung des Vorhofflimmerns nützlich ist, in dem der normale Sinusrhythmus behalten wird und das Auftreten von neuen Vorhofflimmernschüben verhindert oder reduziert wird.

11. Ein Kit von Teilen umfassend mindestens zwei separate Einheitsarzneiformen (A) und (B), wobei
(A) eine Verbindung umfasst, die ausgewählt ist aus
*N*-(2,6-Dimethylphenyl)-2-[4-[2-hydroxy-3-(2-methoxyphenoxy)-propyl]piperazin-1-yl]acetamid,
(3*R*)-1-{(1*R*,2*R*)-2-[2-(3,4-Dimethoxyphenyl)ethoxy]cyclohexyl}pyrrolidin-3-ol,
1-[2-[Bis(4-fluorophenyl)methoxy]ethyl]-4-(3-phenylpropyl)piperazin,
2-Butyl-3-benzofuranyl-4-[2-(diethylamino)-ethoxy]-3,5-diiodphenylketon,
N-{2-Butyl-3-[4-(3-dibutylaminopropoxy)benzoyl]benzofuran-5-yl}methansulfonamid,
N-[4-[2-[2-[4-(Methansulfonamido)phenoxy]ethyl-methylamino]ethyl]phenyl]-methansulfonamid,
N-[4-[4-[Ethyl(heptyl)amino]-1-hydroxybutyl]phenyl]methansulfonamid, und
N-[4-[1-Hydroxy-2-(propan-2-ylamino)ethyl]phenyl]methansulfonamid;
und
(B) einen Hemmer der SK-Kanäle (Calcium-aktivierten Kalium-Kanäle mit kleiner Leitfähigkeit) oder ein pharmazeutisch akzeptables Salz oder Solvat davon umfasst; und wahlweise
(C) Anweisungen zur gleichzeitigen, sequenziellen oder getrennten Verabreichung der Verbindung von (A) und des Hemmers der SK-Kanäle (Calcium-aktivierten Kalium-Kanäle mit kleiner Leitfähigkeit) von B, einem Patienten, der sie braucht.

## Revendications

1. Une combinaison d'un
(i) inhibiteur des canaux potassiques activés par le calcium de basse conductance (SK), ou un sel ou solvat pharmaceutiquement acceptable de celui-ci ; et
(ii) un composé choisi de
*N*-(2,6-diméthylphényl)-2-[4-[2-hydroxy-3-(2-méthoxyphénoxy)-propyl]pipérazin-l-yl]acétamide,
(3*R*)-1-{(1*R*,2*R*)-2-[2-(3,4-diméthoxyphényl)éthoxy]cyclohexyl}pyrrolidin-3-ol,
1-[2-[bis(4-fluorophényl)méthoxy]éthyl]-4-(3-phénylpropyl)pipérazine,
2-butyl-3-benzofuranyl 4-[2-(diéthylamino)-éthoxy]-3,5-diiodophénylcétone,
N-{2-butyl-3-[4-(3-dibutylaminopropoxy)benzoyl]benzofuran-5-yl}méthanesulfonamide,
N-[4-[2-[2-[4-(méthanesulfonamido)phénoxy]éthyl-méthylamino]éthyl]phényl]-méthanesulfonamide,
N-[4-[4-[éthyl(héptyl)amino]-1-hydroxybutyl]phényl]méthanesulfonamide,
N-[4-[1-hydroxy-2-(propan-2-ylamino)éthyl]phényl]méthanesulfonamide, ou un sel ou solvat pharmaceutiquement acceptable de ceux-ci,
pour l'utilisation comme un médicament.

2. La combinaison pour l'utilisation selon la revendication 1 dans laquelle le composé est choisi parmi le dihydrochlorure de ranolazine, l'hydrochlorure de vernakalant, le dihydrochlorure de vanoxérine, l'hydrochlorure de vanoxérine, l'hydrochlorure d'amiodarone, l'hydrochlorure de dronédarone, le dofétilide, le fumarate d'ibutilide, et l'hydrochlorure de sotalol.

3. La combinaison pour l'utilisation selon l'une quelconque des revendications 1-2, dans laquelle le composé est le dihydrochlorure de ranolazine.

4. La combinaison pour l'utilisation selon l'une quelconque des revendications 1-3, dans laquelle l'inhibiteur des canaux potassiques activés par le calcium de basse conductance (SK) est un modulateur négatif de SK.

5. La combinaison pour l'utilisation selon l'une quelconque des revendications 1-3, dans laquelle l'inhibiteur des canaux potassiques activés par le calcium de basse conductance (SK) est un bloqueur des pores des canaux SK.

6. Une composition pharmaceutique comprenant la combinaison selon l'une quelconque des revendications 1-5.

7. La combinaison selon l'une quelconque des revendications 1-5 pour l'utilisation comme un médicament pour le traitement d'une maladie, un trouble ou une affection cardiaque lié(e) à un rythme anormal du cœur choisi(e) parmi l'arythmie cardiaque, l'arythmie atriale, l'arythmie ventriculaire, la fibrillation atriale, la fibrillation ventriculaire, la tachyarythmie, la tachyarythmie atriale, la tachyarythmie ventriculaire, et les bradyarythmies.

8. La combinaison pour l'utilisation selon la revendication 7, dans laquelle la maladie, le trouble ou l'affection cardiaque lié(e) à un rythme anormal du cœur est la fibrillation atriale.

9. La combinaison pour l'utilisation selon la revendication 8 utile pour le traitement de la fibrillation atriale par cardioversion aiguë en rythme sinusal normal.

10. La combinaison pour l'utilisation selon la revendication 8 utile pour le traitement de la fibrillation atriale en maintenant le rythme sinusal normal et en empêchant ou réduisant la survenance d'épisodes nouveaux de fibrillation atriale.

11. Un kit de pièces comprenant au moins deux formes galéniques unitaires distinctes (A) et (B), dans lequel
(A) comprend un composé choisi de
*N*-(2,6-diméthylphényl)-2-[4-[2-hydroxy-3-(2-méthoxyphénoxy)-propyl]pipérazin-1-yl]acétamide,
(3*R*)-1-{(1*R*,2*R*)-2-[2-(3,4-diméthoxyphényl)éthoxy]cyclohexyl}pyrrolidin-3-ol,
1-[2-[bis(4-fluorophényl)méthoxy]éthyl]-4-(3-phénylpropyl)pipérazine,
2-butyl-3-benzofuranyl 4-[2-(diéthylamino)-éthoxy]-3,5-diiodophénylcétone,
N-{2-butyl-3-[4-(3-dibutylaminopropoxy)benzoyl]benzofuran-5-yl}méthanesulfonamide,
N-[4-[2-[2-[4-(méthanesulfonamido)phénoxy]éthyl-méthylamino]éthyl]phényl]-méthanesulfonamide,
N-[4-[4-[éthyl(héptyl)amino]-1-hydroxybutyl]phényl]méthanesulfonamide, et
N-[4-[1-hydroxy-2-(propan-2-ylamino)éthyl]phényl]méthanesulfonamide;
et
(B) comprend un inhibiteur des canaux potassiques activés par le calcium de basse conductance (SK), ou un sel ou solvat pharmaceutiquement acceptable de celui-ci ; et, facultativement,
(C) des instructions pour l'administration simultanée, séquentielle ou séparée du composé de (A) et de l'inhibiteur des canaux potassiques activés par le calcium de basse conductance (SK) de (B) à un patient en ayant besoin.
